(19) Europäisches Patentamt
European Patent Office
Office européen des brevets

(11) **EP 3 568 266 B1**

(12) **FASCICULE DE BREVET EUROPEEN**

(45) Date de publication et mention
de la délivrance du brevet:
**04.10.2023 Bulletin 2023/40**

(21) Numéro de dépôt: **18702756.0**

(22) Date de dépôt: **10.01.2018**

(51) Classification Internationale des Brevets (IPC):
**B25J 9/00** *(2006.01)* **B62D 57/032** *(2006.01)*
**A61H 3/00** *(2006.01)*

(52) Classification Coopérative des Brevets (CPC):
**B25J 9/0006; A61H 3/00; B62D 57/032;**
A61H 2003/007; A61H 2201/165

(86) Numéro de dépôt international:
**PCT/FR2018/050057**

(87) Numéro de publication internationale:
**WO 2018/130784 (19.07.2018 Gazette 2018/29)**

(54) **PROCÉDÉ DE MISE EN MOUVEMENT D'UN EXOSQUELETTE**

VERFAHREN ZUM BEWEGEN EINES EXOSKELETTS

METHOD FOR MOVING AN EXOSKELETON

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **10.01.2017 FR 1750217**

(43) Date de publication de la demande:
**20.11.2019 Bulletin 2019/47**

(73) Titulaire: **Wandercraft**
**75004 Paris (FR)**

(72) Inventeurs:
• **MASSELIN, Matthieu**
**91400 Orsay (FR)**
• **NGUYEN, Kien Cuong**
**91400 Orsay (FR)**

(74) Mandataire: **Regimbeau**
**20, rue de Chazelles**
**75847 Paris Cedex 17 (FR)**

(56) Documents cités:
**FR-A1- 3 034 659**

• **UGURLU BARKAN ET AL: "Lower body
exoskeleton-supported compliant bipedal
walking for paraplegics: How to reduce upper
body effort?", 2014 IEEE INTERNATIONAL
CONFERENCE ON ROBOTICS AND
AUTOMATION (ICRA), IEEE, 31 mai 2014
(2014-05-31), pages 1354-1360, XP032650341,
DOI: 10.1109/ICRA.2014.6907028**
• **MANCHESTER IAN R ET AL: "Real-time planning
with primitives for dynamic walking over uneven
terrain", 2014 IEEE INTERNATIONAL
CONFERENCE ON ROBOTICS AND
AUTOMATION (ICRA), IEEE, 31 mai 2014
(2014-05-31), pages 4639-4646, XP032650038,
DOI: 10.1109/ICRA.2014.6907537**
• **AYUSH AGRAWAL ET AL: "First Steps Towards
Translating HZD Control of Bipedal Robots to
Decentralized Control of Exoskeletons", IEEE
ACCESS, 23 mai 2017 (2017-05-23), pages 1-1,
XP055376624, DOI:
10.1109/ACCESS.2017.2690407**

**Description**

DOMAINE TECHNIQUE GENERAL

La présente invention concerne le domaine des robots de type exosquelette.

Plus précisément, elle concerne un procédé de mise en mouvement d'un exosquelette.

ETAT DE L'ART

Récemment, sont apparus pour les personnes avec des problèmes de mobilité importants comme les paraplégiques des dispositifs de marche assistée appelés exosquelettes, qui sont des dispositifs robotisés externes que l'opérateur (l'utilisateur humain) vient « enfiler » grâce à un système d'attaches qui lie les mouvements de l'exosquelette de ses propres mouvements. Les exosquelettes de membres inférieurs disposent de plusieurs articulations, généralement au moins au niveau des genoux et des hanches, pour reproduire le mouvement de marche. Des actionneurs permettent de mouvoir ces articulations, qui à leur tour font se mouvoir l'opérateur. Un système d'interface permet à l'opérateur de donner des ordres à l'exosquelette, et un système de commande transforme ces ordres en commande pour les actionneurs. Des capteurs viennent généralement compléter le dispositif.

Ces exosquelettes constituent une avancée par rapport aux fauteuils roulant, car ils permettent aux opérateurs de se remettre debout et de marcher. Les exosquelettes ne sont plus limités par les roues et peuvent théoriquement évoluer dans la majorité des environnements non plats : les roues, au contraire des jambes, ne permettent pas de franchir des obstacles importants comme des marches, escaliers, obstacles d'une hauteur trop importante, etc.

Cependant, dans leur usage, aucun de ces exosquelettes ne réalise une marche humaine autonome, i.e. stable et viable sur une large variété de terrains, anthropomorphe, non assistée.

Dans la plupart des cas, ces limitations se matérialisent par l'impossibilité pour le dispositif de gérer l'équilibre ou la direction de marche par lui-même. Ces deux tâches sont alors généralement transférées à l'opérateur, qui les réalise grâce à des béquilles, comme proposé par exemple dans le brevet US7153242 de Rewalk, ou dans la demande US2016038371 de Ekso-Bionics. Le document Ugurlu Barkan et al., (2014): "Lower body exoskeleton-supported compliant bipedal walking for paraplégies: How to reduce upper body effort?" décrit un autre exemple d'algorithme de contrôle d'un exosquelette.

Le brevet EP2231096 de Rex-Bionics décrit le seul exosquelette utilisable sans aide extérieure pour une personne incapable d'assurer sa propre stabilité. Le principe de contrôle, décrit au paragraphe [0122], explicite clairement le besoin de transférer le centre de pression (le point physique correspondant à la résultante des forces de réaction exercées par le sol sur le système) d'une partie du polygone de support (l'enveloppe convexe des points de contact avec le sol) vers une autre partie du polygone de support.

Cette limitation impose une marche extrêmement lente (quelques mètres par minute) avec des pas courts (moins de 30cm), durant laquelle le pied d'appui est constamment en contact plan avec le sol. Le type d'environnement accessible est donc limité, puisque les terrains accidentés sont exclus de fait. De même, le moindre obstacle comme un caillou, un petit objet, génère un risque de déséquilibrer le système s'il pose son pied dessus à un moment donné, et finalement de le faire tomber.

Il serait par conséquent souhaitable de disposer d'un nouveau paradigme de marche pour les exosquelettes qui s'affranchisse des contraintes actuelles, de sorte à proposer une marche rapide, naturelle, et sans risque de chute ou de déséquilibre même en terrain difficile et imprévu.

PRESENTATION DE L'INVENTION

La présente invention se rapporte ainsi selon un premier aspect à un procédé de mise en mouvement d'un exosquelette recevant un opérateur humain, ledit exosquelette présentant une pluralité de degrés de liberté dont au moins un degré de liberté actionné par un actionneur commandé par des moyens de traitement de données et au moins un degré de liberté non actionné, le procédé étant caractérisé en ce qu'il comprend la mise en oeuvre par les moyens de traitement de données d'étapes de :

    (a) Lorsqu'une requête de démarrage est reçue, génération et émission d'une commande à au moins un desdits actionneurs de sorte à placer l'exosquelette dans un état de basculement ;
    (b) dans une base de données stockée dans des moyens de stockage de données de triplets de :

- un jeu de contraintes virtuelles sur lesdits degrés de liberté actionnés, les contraintes virtuelles étant paramétrées par une variable de phase,
- un contrôleur dudit exosquelette capable de générer des commandes desdits actionneurs de sorte à respecter lesdites contraintes virtuelles en mettant en oeuvre au moins une trajectoire stable attractrice dans une variété topologique formée par l'ensemble des n-uplets des valeurs possibles desdits degrés de liberté non-actionnés et de la variable de phase,
- un bassin de stabilité formé par l'ensemble des points d'un hyperplan de ladite variété pour une valeur donnée du paramètre de phase, à partir desquels l'exécution dudit contrôleur permet une convergence vers ladite trajectoire stable attractrice ;

identification d'un jeu de contraintes virtuelles tel que ledit état de basculement est compris dans ledit bassin de stabilité associé à ce jeu de contraintes virtuelles ;

(c) exécution du contrôleur associé au jeu de contraintes virtuelles identifié de sorte à ce que l'exosquelette marche.

Selon d'autres caractéristiques avantageuses et non limitatives :

- l'étape (a) comprend la détermination d'une consigne de vitesse et/ou de direction de marche en fonction desquelles la ladite commande à au moins un desdits actionneurs est générée, l'étape (c) comprenant la vérification que ladite consigne de vitesse et/ou de direction est respectée par la marche en cours dans ladite base de données ;
- le procédé comprend, si ladite consigne de vitesse et/ou de direction n'est pas respectée par la marche en cours, une étape (d) d'identification dans ladite base de données d'un nouveau jeu de contraintes virtuelles tel que l'état actuel de l'exosquelette est compris dans ledit bassin de stabilité associé à ce jeu de contraintes virtuelles ; et la répétition de l'étape (c) ;
- ladite consigne de vitesse et/ou de direction de marche est déterminée en fonction d'une posture dudit opérateur humain ;
- un buste de l'opérateur est équipé d'une pluralité de capteurs de posture, ladite consigne de vitesse et/ou de direction de marche étant déterminée en fonction de la posture dudit buste de l'opérateur mesurée par la pluralité de capteurs ;
- l'état de basculement est un état dans lequel un Point de Moment Zéro, ZMP, n'est pas à l'intérieur d'une surface de sustentation de l'exosquelette ;
- l'étape (c) comprend l'arrêt de l'exosquelette si aucun jeu de contraintes virtuelles acceptable n'est identifié.

Selon un deuxième aspect, l'invention concerne un exosquelette comprenant des moyens de traitement de données et présentant une pluralité de degrés de liberté dont au moins un degré de liberté actionné par un actionneur commandé par les moyens de traitement de données et au moins un degré de liberté non actionné, caractérisé en ce qu'il comprend des moyens de stockage de données stockant une base de données de triplets de :

- un jeu de contraintes virtuelles sur lesdits degrés de liberté actionnés, les contraintes virtuelles étant paramétrées par une variable de phase,
- un contrôleur dudit exosquelette capable de générer des commandes desdits actionneurs de sorte à respecter lesdites contraintes virtuelles en mettant en oeuvre au moins une trajectoire stable attractrice dans une variété topologique formée par l'ensemble des n-uplets des valeurs possibles desdits degrés de liberté non-actionnés et de la variable de phase,

- un bassin de stabilité formé par l'ensemble des points d'un hyperplan de ladite variété pour une valeur donnée du paramètre de phase, à partir desquels l'exécution dudit contrôleur permet une convergence vers ladite trajectoire stable attractrice ;

et en ce que les moyens de traitement de données sont configurés pour mettre en oeuvre :

- Un module de génération et émission d'une commande à au moins un desdits actionneurs de sorte à placer l'exosquelette dans un état de basculement lorsqu'une requête de démarrage est reçue ;
- Un module d'identification dans ladite base de données des moyens de stockage d'un jeu de contraintes virtuelles tel que ledit état de basculement est compris dans ledit bassin de stabilité associé à ce jeu de contraintes virtuelles ;
- Un module d'exécution du contrôleur associé au jeu de contraintes virtuelles identifié de sorte à ce que l'exosquelette marche.

Selon un troisième et un quatrième aspect, l'invention concerne un produit programme d'ordinateur comprenant des instructions de code pour l'exécution d'un procédé mise en mouvement d'un exosquelette selon le premier aspect de l'invention ; et un moyen de stockage lisible par un équipement informatique sur lequel un produit programme d'ordinateur comprend des instructions de code pour l'exécution d'un procédé mise en mouvement d'un exosquelette selon le premier aspect de l'invention.

PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :

- la figure 1 est un schéma d'un exosquelette (de type exosquelette) pour la mise en oeuvre du procédé selon l'invention ;
- La figure 2 représente un exemple d'évolution d'une variable de phase, et d'évolution d'un degré de liberté actionné en fonction de cette variable de phase ;
- la figure 3 représente schématiquement la variété de la dynamique des zéros hybride et le bassin d'attraction d'une trajectoire cyclique ;
- la figure 4 est un diagramme illustrant un mode de réalisation préféré du procédé selon l'invention.

DESCRIPTION DETAILLEE

*Marche pied à plat*

La marche humaine « naturelle » se caractérise par une succession de phases au cours desquelles les pieds peuvent être à plat au sol, en l'air, ou en train de rouler sur le sol. Cette capacité à dérouler le pied est primordiale pour la marche car elle permet de faire des pas plus grands et permet une stabilité sur une grande variété de terrains.

Or les exosquelettes dits de première génération décrits récemment n'ont pas de pied actionné ou gardent le pied d'appui au sol.

Réaliser ce déroulé est en effet complexe pour des robots humanoïdes bipèdes ou des dispositifs robotisés. Lorsque le centre de pression atteint la limite du polygone de support, le système commence à rouler autour de ce point, et n'est donc plus en équilibre statique.

Dans le cas de la marche, le déroulé du pied implique une perte de contact partielle avec le sol au niveau du pied d'appui, avec plusieurs conséquences :

- le polygone de support (la surface de sustentation) est réduit, potentiellement à un point, rendant difficile voire impossible de maintenir le centre de pression à l'intérieur du polygone de support ;
- le système est dans une situation de sous-actionnement, c'est-à-dire qu'il ne peut plus agir sur l'ensemble de ses degrés de liberté. Tous les mouvements ne sont plus alors possibles.

Dans une telle situation, les formalismes classiques de la marche à pieds plats tels que décrits dans le document Kajita S., K. F. (2003). Biped Walking pattern génération by using preview control of Zero-Moment Point. ICRA, (pp. 1620-1626), ou le principe décrit dans le brevet Rex-Bionics EP2231096 ne peuvent plus fonctionner.

Une idée naturelle est d'amener la jambe balançant devant et de poser le deuxième pied par terre pour retrouver un polygone de support et l'équilibre, ceci alors que le système est en libre rotation autour du pied d'appui, en quelque sorte en train de « tomber ». On parle alors de marche dynamique, puisque le corps passe par une succession de postures instables, mais uniquement de façon transitoire (si on « arrêtait » la personne en plein vol elle tomberait).

Dans cette approche de la marche dynamique, amener le pied balançant rapidement dans une position qui rétablisse au moins brièvement l'équilibre est compliqué. En effet, si on fait suivre à ce pied une trajectoire paramétrée en temps pré-calculée, ce pied risque de frapper le sol trop tôt ou trop tard dû au comportement incontrôlable du système sous-actionné même soumis à de légères perturbations (on ne peut pas corriger une trajectoire qui dévierait légèrement de ce qui a été planifié). Cela peut générer de l'inconfort à l'opérateur, le déséquilibrer voir même le faire tomber, y compris sur des terrains simple.

C'est pour cela que tous les exosquelettes de première génération (et nombre de robots humanoïdes) essayent d'éviter ce genre de situation en gardant le pied d'appui à plat, avec pour conséquences les limitations susmentionnées sur la vitesse de marche, la longueur des pas, le type de terrain admissible et la stabilité générale de la marche.

*Architecture*

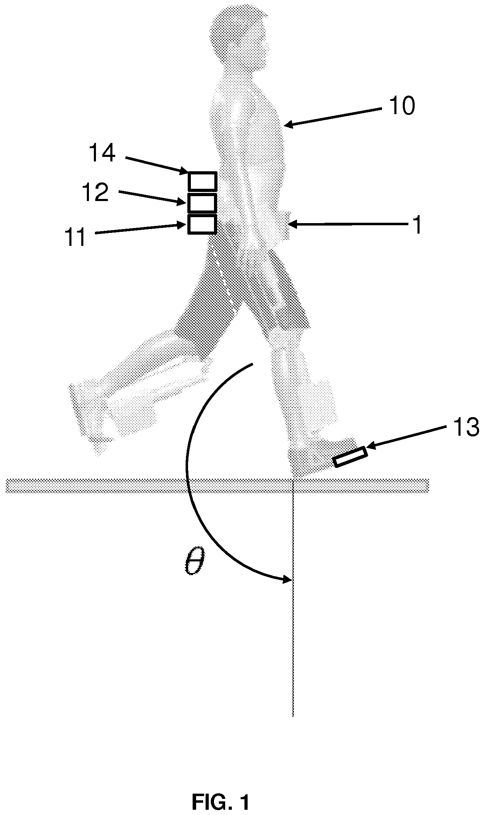

En référence à la **figure 1,** le présent procédé est un procédé de marche d'un exosquelette 1, i.e. un système mécanique articulé de type dispositif robotisé bipède, actionné et commandé, pourvu de deux jambes, accueillant plus précisément un opérateur humain présentant ses membres inférieurs chacun solidaires d'une jambe de l'exosquelette 1 (notamment grâce à des sangles). Il peut ainsi être un robot plus ou moins humanoïde. Par « marche », on entend ici la mise en mouvement du dispositif robotisé 1, qui se traduit en pratique par en appui alternatif sur les jambes, en position debout, de sorte à produire un déplacement.

L'exosquelette 1 présente une pluralité de degrés de liberté, c'est-à-dire d'articulations déformables (généralement via une rotation) c'est-à-dire mobiles les unes par rapport aux autres, qui sont chacun soit « actionné », soit « non-actionné ».

Un degré de liberté actionné désigne une articulation pourvue d'un actionneur commandé par des moyens de traitement de données 11, c'est-à-dire que ce degré de liberté est contrôlé et que l'on peut agir dessus. Au contraire un degré de liberté non actionné désigne une articulation dépourvue d'actionneur, c'est-à-dire que ce degré de liberté suit sa propre dynamique et que les moyens de traitement de données 11 n'ont pas de contrôle direct dessus (mais a priori un contrôle indirect via les autres degrés de liberté actionnés). Dans l'exemple de la figure 1, le contact talon-sol est ponctuel, et l'exosquelette 1 est ainsi libre en rotation par rapport à ce point de contact. L'angle entre l'axe talon-hanche et la verticale constitue alors un degré de liberté non-actionné.

Le présent exosquelette comprend naturellement au moins un degré de liberté actionné, préférentiellement une pluralité, et également au moins un degré de liberté non actionné, c'est-à-dire qu'il est dit « sous-actionné », comme évoqué précédemment. On appelle degré de sous-actionnement le nombre de degrés de liberté non-actionnés.

Les moyens de traitement de données 11 désignent un équipement informatique (typiquement un processeur, soit externe si l'exosquelette 1 est « télécommandé » mais préférentiellement embarqué dans l'exosquelette 1) adapté pour traiter des instructions et générer des commandes à destination des différents actionneurs. Ces derniers peuvent être électriques, hydrauliques, etc.

La présente demande ne sera limitée à aucune architecture d'exosquelette 1, et on prendra l'exemple tel que décrit dans les demandes WO2015140352 et WO2015140353.

Ainsi, de façon préférée et conformément à ces demandes, l'exosquelette 1 comprend sur chaque jambe une structure de pied comprenant un plan de support sur lequel un pied d'une jambe de la personne portant l'exosquelette peut venir en appui lorsque le pied est à plat.

Ce plan de support comprend une plate-forme avant et une plate-forme arrière, telles qu'une liaison pivot pied relie la plate-forme avant à la plate-forme arrière, en constituant un degré de liberté non actionné.

L'homme du métier saura toutefois adapter le présent procédé à toute autre architecture mécanique.

*Dynamique des Zéros Hybride*

De façon traditionnelle, les trajectoires / évolutions de chaque degré de liberté sont exprimées en fonction du temps. La « dynamique » du système est définie par une fonction $f : \chi \times \mathcal{U} \times \mathbb{R}^+ \mapsto \chi$ et un point de départ $\xi \in \chi$ la fonction $f$ s'écrivant $\dot{x}_t = f(x_t, u_t, t), x_0 = \xi$, $\chi$ étant l'espace d'état de l'exosquelette 1 et $\mathcal{U}$ l'espace de contrôle.

Au contraire, en méthode dite des « contraintes virtuelles », le principe est de définir pour une sélection des degrés de liberté actionnés une trajectoire paramétrée par un paramètre d'évolution non pas en temps, mais en fonction directement de la configuration, ce paramètre étant nommé variable de phase. Un exemple d'une telle variable de phase est représenté sur la figure 1, il s'agit de l'angle entre l'axe talon-hanche et la verticale qui constitue alors un degré de liberté non-actionné mentionné ci-avant.

La variable de phase permet de définir « l'avancement » d'un pas. Plus précisément, à chaque pas, la variable de phase passe continûment d'une valeur initiale à une valeur finale, avant de se voir réaffecter la valeur initiale : c'est le début du pas suivant. Pour faciliter les choses, on peut normaliser la valeur du paramètre de phase entre 0 et 1.

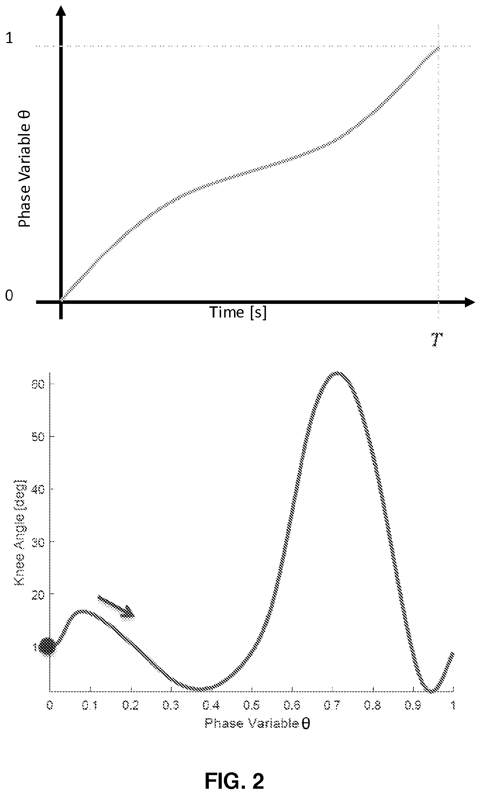

A chaque valeur du paramètre d'évolution correspond une valeur des degrés de liberté actionnés que le système doit s'efforcer de suivre : ce sont ces relations (une pour chaque degré de liberté actionné que l'on souhaite contrôler de cette manière) qu'on nomme contraintes virtuelles. La **Figure 2** montre le fonctionnement des contraintes virtuelles pour une articulation, le genou.

Si le système suit exactement cette trajectoire pour les degrés de liberté sur lesquels on peut ou l'on veut agir, en d'autres termes si les contraintes virtuelles sont respectées pour ces degrés de liberté, alors l'évolution du système est totalement déterminée par celle des degrés de liberté non-actionnés qui suivent leur propre dynamique.

Cette dynamique est appelée « Dynamique des Zéros Hybride », ou HZD (Hybrid Zéro Dynamics), car :

- elle est dite « Zéro » puisqu'elle correspond aux degrés sur lesquels la commande ne peut/veut pas agir, i.e. la commande vaut 0 ;
- elle est dite « Hybride » car l'impact du pied sur le sol impose des phases instantanées discontinues qui entrecoupent les phases continues.

Cette Dynamique des Zéros Hybride dépend des contraintes virtuelles choisies, et un bon choix de celles-ci peut amener cette dynamique à contenir une « orbite » périodique attractive, i.e. une trajectoire vers laquelle le système est attiré naturellement.

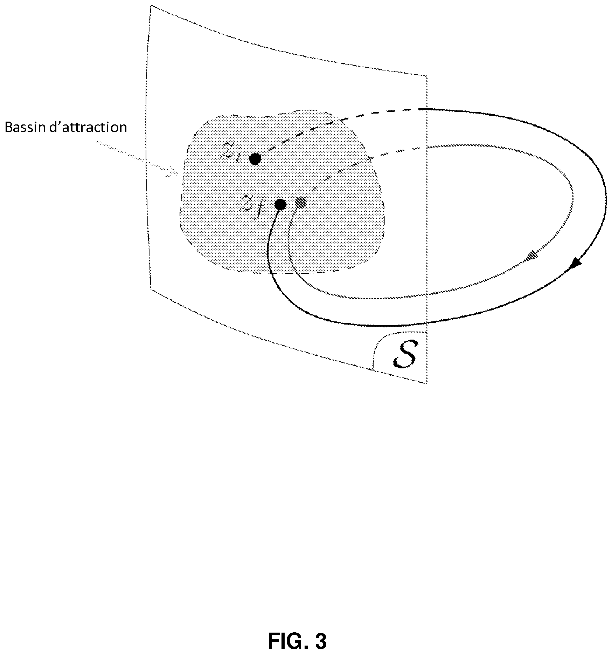

Plus visuellement, et comme illustré par la **figure 3,** pour un jeu de contraintes virtuelles donné l'espace d'état « contraint » est une variété topologique de la dynamique des zéros hybride dans laquelle chaque point est défini par un vecteur des valeurs des degrés de liberté non-actionnés et de la valeur du paramètre de phase (et le cas échéant leur dérivées). On comprend que cette variété n'est pas nécessairement de dimension 3 comme c'est le cas dans la figure 3, il s'agit uniquement d'un exemple facilitant la compréhension du phénomène et on définira que la variété est de dimension n.

Chaque valeur du paramètre de phase définit un hyperplan (de dimension n-1) de la variété contenant tous les n-1-uplets de valeurs possibles des degrés de libertés non-actionnés pour cette valeur du paramètre de phase.

L'un de ces hyperplans, représenté par la figure 3, est l'ensemble $\mathcal{S}$ appelé « switching surface », qui correspond à la frontière entre deux cycles, i.e. le point où la variable de phase revient de la valeur finale à la valeur initiale : lors d'un pas, on part d'un état de $\mathcal{S}$, on applique la dynamique tant que

$$x \notin \mathcal{S}$$

en formant une orbite jusqu'à revenir à un point de $\mathcal{S}$.

Ainsi on passe d'un système de « continu en dimension *n* » à « discret en dimension *n*-1 » en raisonnant par cycle.

Si le point de départ et le point d'arrivée sont les mêmes (« point fixe »), on obtient une orbite périodique dans la variété de la dynamique des zéros hybride comme représenté par la figure 3.

Pour qu'une orbite périodique soit dite attractive, il faut en plus qu'en partant d'un point distinct du point fixe, on se rapproche à l'itération suivante du point fixe (par opposition à des orbites périodiques instables dans lesquelles on diverge dès que l'on s'éloigne un tant soit peu du point fixe).

On peut ainsi définir un bassin d'attraction, c'est-à-dire un sous espace de $\mathcal{S}$ comprenant l'ensemble desdits points distincts du point fixes se rapproche à l'itération suivante du point fixe, i.e. dans lequel toute trajectoire commençant dans ce bassin approche de plus en plus l'orbite périodique attractive après chaque cycle de marche (trajectoire $z_i \rightarrow z_f$). Plus précisément, le système converge pour un point de départ dans le bassin, et diverge pour un point de départ hors du bassin (c'est-à-dire finit par chuter).

Il est alors possible de designer un contrôleur de type HZD (on verra comment plus loin) qui contraint le système à respecter les contraintes virtuelles et maximise le caractère attractif du cycle pour les degrés de libertés sous-actionnés. Cela permet d'abord de s'assurer que l'exosquelette 1 sera par exemple bien capable d'amener son pied balançant vers l'avant et d'atterrir sur le sol ni trop tôt ni trop tard malgré le caractère sous-actionné. En plus, même en présence de perturbations, tant que l'état du système reste dans le bassin d'attraction, les degrés de libertés sous-actionnés convergent naturellement vers l'orbite périodique et le système revient vers un cycle de marche prévue après quelques pas et la vitesse de marche est donc assurée.

Par ailleurs, ce concept permet de gérer aussi bien les phases durant lesquelles le pied d'appui est en contact avec le sol, en rotation autour de l'avant du pied ou durant la phase d'arrivée au sol et de rotation autour de l'arrière du pied. A chaque fois, il est possible de trouver des contraintes virtuelles pour les degrés de libertés actionnés qui génère une Dynamique des Zéros Hybride pour les autres degrés de liberté dans laquelle un cycle attracteur existe.

On peut générer autant de jeu de contraintes que nécessaires, correspondant à des longueurs de pas, des vitesses de marche, et des directions différentes.

*Procédé*

Si la notion de contrôleur de type HZD est déjà connue et permet de générer des trajectoires stables, ces dernières sont jusqu'ici cycliques et à vitesse initiale non nulle. Cette notion est donc inapplicable aux systèmes partant de l'arrêt, et a fortiori de façon non exactement reproductible (à cause de la présence de l'opérateur humain) que sont les exosquelettes,

Le présent procédé de mise en mouvement d'un exosquelette 1 résout astucieusement ces difficultés en fournissant un moyen de se placer à l'intérieur du bassin d'attraction.

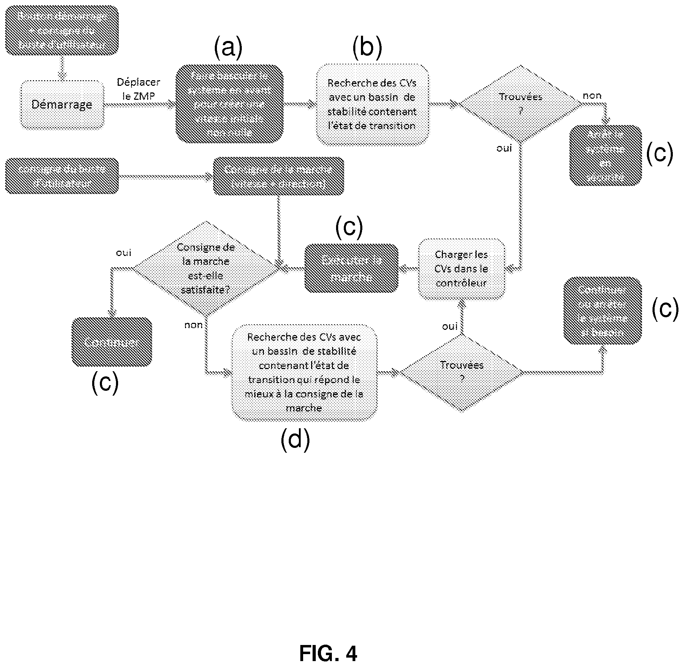

En pratique, et comme illustré par la **figure 4,** le présent procédé commence par une étape (a) de mise de l'exosquelette 1 dans un état de basculement. Plus précisément, lorsqu'une requête de démarrage est reçue, les moyens de traitement de données génèrent et émettent une commande à au moins un desdits actionneurs de sorte à placer sciemment l'exosquelette 1 dans cet état de basculement.

Par état de basculement, on entend avantageusement un état dans lequel un Point de Moment Zéro, ZMP (« Zéro Moment Point »), n'est pas à l'intérieur d'une surface de sustentation de l'exosquelette 1. Le ZMP désigne plus précisément le point où le moment des forces de contact a deux de ses trois coordonnées nulles (purement vertical).

Ainsi, la mise en basculement correspond préférentiellement a un déplacement volontaire du ZMP de sorte à « faire sortir » le ZMP de la surface de sustentation, via un algorithme de démarrage. Il s'agit donc d'une situation de « chute » qu'on cherchait jusqu'à présente toujours à éviter à tout prix. Cet algorithme de démarrage peut se baser sur des mouvements paramétrés en temps pré-calculés et testés en amont ou sur des mouvements référencés capteurs, par exemple, des moyens de détection de l'impact des pieds au sol 13 et/ou des moyens de mesure inertielle 14 équipés sur l'exosquelette 1.

Avant cela, l'étape (a) comprend avantageusement la détermination d'une consigne de vitesse et/ou de direction de marche en fonction desquelles la ladite commande à au moins un desdits actionneurs est générée.

En effet, si l'exosquelette 1 est un exosquelette recevant un opérateur humain, c'est la posture dudit opérateur humain qui détermine ladite consigne de vitesse et/ou de direction de marche (contrairement au cas d'un robot normal qui peut directement recevoir une requête de démarrage comprenant une consigne de vitesse et/ou de direction de marche).

Pour cela, l'opérateur peut être muni d'un gilet de capteurs 10 permettant de détecter la configuration de son buste (orientation de celui-ci). La direction dans laquelle l'opérateur oriente son buste est celle dans laquelle il souhaite marcher et la vitesse est donnée par l'intensité avec laquelle il met son buste en avant (à quel point il se penche). La requête de démarrage peut correspondre à l'appui par l'opérateur sur un bouton (ou une posture particulière) signifiant son intention de se mettre en marche et donc ordonnant aux moyens de traitement de données de déterminer la consigne de direction et/ou de vitesse

Dans une étape (b) principale, est identifié un jeu de contraintes virtuelles tel que ledit état de basculement est compris dans le bassin de stabilité associé à ce jeu de contraintes virtuelles dans une base de données.

En effet, le présent procédé propose l'utilisation d'une base de données (appelée bibliothèque de contrôle) stockée dans des moyens de stockage de données 12 (une mémoire connectée aux moyens de traitement de données 11 de triplets de :

- un jeu de contraintes virtuelles sur lesdits degrés de liberté actionnés, les contraintes virtuelles étant paramétrées par une variable de phase (auquel correspond comme expliqué un cycle attracteur pour les degrés de liberté sous-actionnés, définissant ainsi une trajectoire complète pour le système),

- un contrôleur (HZD) dudit exosquelette 1 capable de générer des commandes desdits actionneurs de sorte à respecter lesdites contraintes virtuelles en mettant en oeuvre au moins une trajectoire stable attractrice dans ladite variété topologique formée par l'ensemble des n-uplets des valeurs possibles desdits degrés de liberté non-actionnés et de la variable de phase,

- un bassin de stabilité formé par l'ensemble des points de l'hyperplan $S$ de ladite variété pour une valeur donnée du paramètre de phase, à partir desquels l'exécution dudit contrôleur permet une convergence vers ladite trajectoire stable attractrice (i.e. une absence de divergence) ;

Les différents jeux de contraintes virtuelles correspondent typiquement et comme expliqué à diverses longueurs de pas, vitesses de marche, et directions différentes, diverses façons de marcher, etc. L'homme du métier sait les générer.

Pour chaque contrôleur HZD il a pu être vérifié en simulation voire en pratique qu'il rendait la trajectoire associée stable i.e. qu'il permettait même en présence de perturbations de se ramener rapidement à cette trajectoire et ce même sur les degrés de libertés sous-actionnés.

On comprendra que les bassins de stabilité peuvent être également déterminés par simulation (pour chaque point de $S$ on teste si le système diverge ou converge, et on identifie l'ensemble des points tels qu'il converge).

Cette solution s'avère particulièrement efficace, car toute la complexité de la marche dynamique est réalisée en amont. En fonctionnement, l'exosquelette n'a plus qu'à rechercher dans la bibliothèque de contrôle un jeu de contraintes définissant une trajectoire complète pour le système et stable dans l'état actuel de basculement, et il est garanti que le contrôleur HZD saura réaliser une marche sure malgré d'éventuelles perturbations.

Si aucun jeu de contraintes virtuelles acceptable n'est identifié (i.e. pour chaque jeu de contraintes virtuelles l'état de basculement est hors du bassin de stabilité), dans une étape (c) l'arrêt de l'exosquelette est commandé pour garantir la sécurité de l'opérateur.

Si un jeu de contraintes virtuelles acceptable est identifié, l'étape (c) voit l'exécution du contrôleur associé au jeu de contraintes virtuelles identifié de sorte à ce que l'exosquelette 1 marche, i.e. réalise au moins un cycle (un pas).

Si a été déterminé une consigne de vitesse et/ou de direction de marche, l'étape (c) comprend alors avantageusement la vérification préalable que ladite consigne de vitesse et/ou de direction est respectée par la marche en cours dans ladite base de données, i.e.

qu'une erreur entre la vitesse et/ou direction de la marche en cours et ladite consigne de vitesse et/ou de direction est inférieure à un seuil prédéterminé.

En effet, il faut que le jeu de contraintes identifié permette non seulement une trajectoire stable, mais également qu'il soit conformes aux consignes de l'opérateur.

Si ladite consigne de vitesse et/ou de direction est respectée par la marche en cours (i.e. l'erreur est en dessous du seuil), celle-ci est continuée.

Au contraire, si ladite consigne de vitesse et/ou de direction n'est pas respectée par la marche en cours (i.e. l'erreur devient trop grande), le procédé comprend avantageusement une étape (d) d'identification dans ladite base de données d'un nouveau jeu de contraintes virtuelles tel que l'état actuel de l'exosquelette 1 est compris dans ledit bassin de stabilité associé à ce jeu de contraintes virtuelles ; et la répétition de l'étape (c), i.e. le chargement et l'exécution d'un nouveau contrôleur HZD.

L'enchainement des étapes (c) et (d) peut alors être répété en boucle, car les consignes de vitesse et/ou de direction peuvent varier à tout moment si l'opérateur le désire : si les consignes changent le jeu actuel peut devenir inadapté et nécessiter l'identification d'un nouveau jeu de contraintes dans la base de données.

A noter qu'à chaque répétition de l'étape (c), elle peut comprendre l'arrêt de l'exosquelette 1 si aucun jeu de contraintes virtuelles acceptable n'est identifié.

*Equipements et système*

Selon un deuxième aspect, l'invention concerne l'exosquelette 1, en particulier de type exosquelette, pour la mise en oeuvre du procédé selon le premier aspect.

Comme expliqué, l'exosquelette 1 comprend des moyens de traitement de données 11 et des moyens de stockage de données 12 (éventuellement externes), et si nécessaire des moyens de mesure inertielle 14 (centrale à inertie) et/ou des moyens pour détecter l'impact des pieds au sol 13 (capteurs de contact ou éventuellement capteurs de pression).

Il présente une pluralité de degrés de liberté dont au moins un degré de liberté actionné par un actionneur commandé par les moyens de traitement de données 11 et au moins un degré de liberté non actionné.

Les moyens de stockage de données 12 stockent une base de données de triplets constitués de :

- un jeu de contraintes virtuelles sur lesdits degrés de liberté actionnés, les contraintes virtuelles étant paramétrées par une variable de phase,

- un contrôleur dudit exosquelette 1 capable de générer des commandes desdits actionneurs de sorte à respecter lesdites contraintes virtuelles en mettant en oeuvre au moins une trajectoire stable attractrice dans une variété topologique formée par l'ensemble des n-uplets des valeurs possibles desdits degrés de liberté non-actionnés et de la variable de phase,
- un bassin de stabilité formé par l'ensemble des points d'un hyperplan de ladite variété pour une valeur donnée du paramètre de phase, à partir desquels l'exécution dudit contrôleur permet une convergence vers ladite trajectoire stable attractrice ;

Et les moyens de traitement de données 11 sont configurés pour mettre en oeuvre :

- Un module de génération et émission d'une commande à au moins un desdits actionneurs de sorte à placer l'exosquelette 1 dans un état de basculement lorsqu'une requête de démarrage est reçue (et le cas échéant de détermination d'une consigne de vitesse et/ou de direction) ;
- Un module d'identification dans ladite base de données des moyens de stockage 12 d'un jeu de contraintes virtuelles tel que ledit état de basculement est compris dans ledit bassin de stabilité associé à ce jeu de contraintes virtuelles (et tel que ladite consigne de vitesse et/ou de direction est respectée) ;
- Un module d'exécution du contrôleur associé au jeu de contraintes virtuelles identifié de sorte à ce que l'exosquelette 1 marche (ou de mise en arrêt de l'exosquelette 1 si aucun jeu de contraintes virtuelles acceptables n'est déterminé).

*Produit programme d'ordinateur*

Selon un troisième et un quatrième aspects, l'invention concerne un produit programme d'ordinateur comprenant des instructions de code pour l'exécution (sur les moyens de traitement 11) d'un procédé de mise en mouvement d'un exosquelette 1 selon le premier aspect de l'invention, ainsi que des moyens de stockage lisibles par un équipement informatique (par exemple les moyens de stockage de données 12) sur lequel on trouve ce produit programme d'ordinateur.

**Revendications**

1. Procédé de mise en mouvement d'un exosquelette (1) recevant un opérateur humain, ledit exosquelette (1) présentant une pluralité de degrés de liberté dont au moins un degré de liberté actionné par un actionneur commandé par des moyens de traitement de données (11) et au moins un degré de liberté non actionné, le procédé étant **caractérisé en ce qu'**il comprend la mise en oeuvre par les moyens de traitement de données (11) d'étapes de :

   (a) Lorsqu'une requête de démarrage est reçue, génération et émission d'une commande à au moins un desdits actionneurs de sorte à placer l'exosquelette (1) dans un état de basculement ;
   (b) dans une base de données stockée dans des moyens de stockage de données (12) de triplets de :

   - un jeu de contraintes virtuelles sur lesdits degrés de liberté actionnés, les contraintes virtuelles étant paramétrées par une variable de phase,
   - un contrôleur dudit exosquelette (1) capable de générer des commandes desdits actionneurs de sorte à respecter lesdites contraintes virtuelles en mettant en oeuvre au moins une trajectoire stable attractrice dans une variété topologique formée par l'ensemble des n-uplets des valeurs possibles desdits degrés de liberté non-actionnés et de la variable de phase,
   - un bassin de stabilité formé par l'ensemble des points d'un hyperplan de ladite variété pour une valeur donnée du paramètre de phase, à partir desquels l'exécution dudit contrôleur permet une convergence vers ladite trajectoire stable attractrice ;

   identification d'un jeu de contraintes virtuelles tel que ledit état de basculement est compris dans ledit bassin de stabilité associé à ce jeu de contraintes virtuelles ;
   (c) exécution du contrôleur associé au jeu de contraintes virtuelles identifié de sorte à ce que l'exosquelette (1) marche.

2. Procédé selon la revendication 1, dans lequel l'étape (a) comprend la détermination d'une consigne de vitesse et/ou de direction de marche en fonction desquelles la ladite commande à au moins un desdits actionneurs est générée, l'étape (c) comprenant la vérification que ladite consigne de vitesse et/ou de direction est respectée par la marche en cours dans ladite base de données.

3. Procédé selon la revendication 2, comprenant, si ladite consigne de vitesse et/ou de direction n'est pas respectée par la marche en cours, une étape (d) d'identification dans ladite base de données d'un nouveau jeu de contraintes virtuelles tel que l'état actuel de l'exosquelette (1) est compris dans ledit bassin de stabilité associé à ce jeu de contraintes virtuelles ; et la répétition de l'étape (c).

4. Procédé selon l'une des revendications 2 et 3, dans lequel ladite consigne de vitesse et/ou de direction de marche est déterminée en fonction d'une posture dudit opérateur humain.

5. Procédé selon la revendication 4, dans lequel un buste de l'opérateur est équipé d'une pluralité de capteurs de posture, ladite consigne de vitesse et/ou de direction de marche étant déterminée en fonction de la posture dudit buste de l'opérateur mesurée par la pluralité de capteurs.

6. Procédé selon l'une des revendications 1 à 5, dans lequel l'état de basculement est un état dans lequel un Point de Moment Zéro, ZMP, n'est pas à l'intérieur d'une surface de sustentation de l'exosquelette (1).

7. Procédé selon l'une des revendications 1 à 6, dans lequel l'étape (c) comprend l'arrêt de l'exosquelette (1) si aucun jeu de contraintes virtuelles acceptable n'est identifié.

8. Exosquelette (1) recevant un opérateur humain, comprenant des moyens de traitement de données (11) et présentant une pluralité de degrés de liberté dont au moins un degré de liberté actionné par un actionneur commandé par les moyens de traitement de données (11) et au moins un degré de liberté non actionné, **caractérisé en ce qu'**il comprend des moyens de stockage de données (12) stockant une base de données de triplets de :

- un jeu de contraintes virtuelles sur lesdits degrés de liberté actionnés, les contraintes virtuelles étant paramétrées par une variable de phase,
- un contrôleur dudit exosquelette (1) capable de générer des commandes desdits actionneurs de sorte à respecter lesdites contraintes virtuelles en mettant en oeuvre au moins une trajectoire stable attractrice dans une variété topologique formée par l'ensemble des n-uplets des valeurs possibles desdits degrés de liberté non-actionnés et de la variable de phase,
- un bassin de stabilité formé par l'ensemble des points d'un hyperplan de ladite variété pour une valeur donnée du paramètre de phase, à partir desquels l'exécution dudit contrôleur permet une convergence vers ladite trajectoire stable attractrice ;

et **en ce que** les moyens de traitement de données (11) sont configurés pour mettre en oeuvre :

- Un module de génération et émission d'une commande à au moins un desdits actionneurs de sorte à placer l'exosquelette (1) dans un état de basculement lorsqu'une requête de démarrage est reçue ;
- Un module d'identification dans ladite base de données des moyens de stockage (12) d'un jeu de contraintes virtuelles tel que ledit état de basculement est compris dans ledit bassin de stabilité associé à ce jeu de contraintes virtuelles ;
- Un module d'exécution du contrôleur associé au jeu de contraintes virtuelles identifié de sorte à ce que l'exosquelette (1) marche.

9. Produit programme d'ordinateur comprenant des instructions de code pour l'exécution d'un procédé de mise en mouvement d'un exosquelette (1) selon l'une des revendications 1 à 7, lorsque ledit programme est exécuté sur un ordinateur.

10. Moyen de stockage lisible par un équipement informatique sur lequel un produit programme d'ordinateur comprend des instructions de code pour l'exécution d'un procédé de mise en mouvement d'un exosquelette (1) selon l'une des revendications 1 à 7.

**Patentansprüche**

1. Verfahren zum Bewegen eines Exoskeletts (1), das einen menschlichen Bediener aufnimmt, wobei das Exoskelett (1) eine Vielzahl von Freiheitsgraden aufweist, einschließlich mindestens eines Freiheitsgrads, der durch einen Aktuator betätigt wird, der durch ein Datenverarbeitungsmittel (11) gesteuert wird, und mindestens eines nicht betätigten Freiheitsgrads, wobei das Verfahren **dadurch gekennzeichnet ist, dass** es das Ausführen der folgenden Schritte durch das Datenverarbeitungsmittel (11) umfasst:

(a) wenn eine Startanforderung empfangen wird, Erzeugen und Aussenden eines Befehls an mindestens einen der Aktoren, um das Exoskelett (1) in einen Kippzustand zu versetzen;
(b) in einer Datenbank, die in einem Datenspeichermittel (12) gespeichert ist, mit Tripletts von:

- einem Satz virtueller Bindungen für die betätigten Freiheitsgrade, wobei die virtuellen Bindungen durch eine Phasenvariable parametrisiert sind,
- einer Steuerung des Exoskeletts (1), die in der Lage ist, Befehle für die Aktuatoren zu erzeugen, um die virtuellen Bindungen zu erfüllen, indem sie mindestens eine anziehende stabile Trajektorie in einer topologischen Mannigfaltigkeit implementiert, die durch alle n-Tupel der möglichen Werte für die nicht betätigten Freiheitsgrade und die Phasenvariable gebildet wird,
- einem Stabilitätspool, der durch alle Punkte einer Hyperebene der Mannigfaltigkeit für einen gegebenen Wert des Phasenparameters gebildet wird, von dem aus die Ausführung der Steuerung eine Konvergenz in Richtung der anziehenden stabilen Trajektorie ermöglicht;

Identifizieren eines Satzes virtueller Bindungen, so dass der Kippzustand in dem Stabilitätspool enthalten ist, das diesem Satz virtueller Bindungen verbunden ist;

(c) Ausführen der Steuerung, die mit dem identifizierten Satz virtueller Bindungen verbunden ist, so dass das Exoskelett (1) funktioniert.

2. Verfahren nach Anspruch 1, wobei der Schritt (a) das Bestimmen eines Laufgeschwindigkeits- und/oder Richtungssollwerts als eine Funktion umfasst, von der der Befehl an mindestens einen der Aktuatoren erzeugt wird, wobei der Schritt (c) das Überprüfen umfasst, dass der Laufgeschwindigkeits- und/oder Richtungssollwert durch den aktuellen Gang in der Datenbank eingehalten wird.

3. Verfahren nach Anspruch 2, das, wenn der Laufgeschwindigkeits- und/oder Richtungssollwert durch den aktuellen Gang nicht eingehalten wird, einen Schritt (d) des Identifizierens eines neuen Satzes virtueller Bindungen in der Datenbank umfasst, so dass der aktuelle Zustand des Exoskeletts (1) in den Stabilitätspool aufgenommen wird, der mit diesem Satz virtueller Bindungen verbunden ist; und das Wiederholen von Schritt (c).

4. Verfahren nach einem der Ansprüche 2 und 3, wobei der Laufgeschwindigkeits- und/oder Richtungssollwert in Abhängigkeit von einer Körperhaltung des menschlichen Bedieners bestimmt wird.

5. Verfahren nach Anspruch 4, wobei einer Oberkörper des Bedieners mit einer Vielzahl von Haltungssensoren ausgestattet wird, wobei der Laufgeschwindigkeits- und/oder Richtungssollwert in Abhängigkeit von der durch die Vielzahl von Sensoren gemessenen Haltung des Oberkörpers des Bedieners bestimmt wird.

6. Verfahren nach einem der Ansprüche 1 bis 5, wobei der Kippzustand ein Zustand ist, in dem ein Nullmomentpunkt, ZMP, nicht innerhalb einer Auftriebsfläche für das Exoskelett (1) liegt.

7. Verfahren nach einem der Ansprüche 1 bis 6, wobei der Schritt (c) das Anhalten des Exoskeletts (1) umfasst, wenn kein akzeptabler Satz virtueller Bindungen identifiziert wird.

8. Exoskelett (1), das einen menschlichen Bediener aufnimmt, ein Datenverarbeitungsmittel (11) umfasst und eine Vielzahl von Freiheitsgraden aufweist, einschließlich mindestens eines Freiheitsgrads, der durch einen Aktuator betätigt wird, der durch das Datenverarbeitungsmittel (11) gesteuert wird, und mindestens eines nicht betätigten Freiheitsgrads, **dadurch gekennzeichnet, dass** es ein Datenspeichermittel (12) umfasst, das eine Datenbank mit Tripletts von Folgendem speichert:

- einem Satz virtueller Bindungen für die betätigten Freiheitsgrade, wobei die virtuellen Bindungen durch eine Phasenvariable parametrisiert sind,
- einer Steuerung des Exoskeletts (1), die in der Lage ist, Befehle für die Aktuatoren zu erzeugen, um die virtuellen Bindungen zu erfüllen, indem sie mindestens eine anziehende stabile Trajektorie in einer topologischen Mannigfaltigkeit implementiert, die durch alle n-Tupel der möglichen Werte für die nicht betätigten Freiheitsgrade und die Phasenvariable gebildet wird,
- einem Stabilitätspool, der durch alle Punkte einer Hyperebene der Mannigfaltigkeit für einen gegebenen Wert des Phasenparameters gebildet wird, von dem aus die Ausführung der Steuerung eine Konvergenz in Richtung der anziehenden stabilen Trajektorie ermöglicht; und dadurch, dass das Datenverarbeitungsmittel (11) dazu konfiguriert ist, Folgendes zu implementieren:

- ein Modul zum Erzeugen und Aussenden eines Befehls an mindestens einen der Aktuatoren, um das Exoskelett (1) in einen Kippzustand zu versetzen, wenn eine Startanforderung empfangen wird;
- ein Modul zum Identifizieren eines Satzes virtueller Bindungen in der Datenbank des Speichermittels (12), so dass der Kippzustand in dem Stabilitätspool enthalten wird, der mit diesem Satz virtueller Bindungen verbunden ist;
- ein Modul zum Ausführen der Steuerung, die mit dem identifizierten Satz virtueller Bindungen verbunden ist, so dass das Exoskelett (1) funktioniert.

9. Computerprogrammprodukt, umfassend Codeanweisungen zum Ausführen eines Verfahrens zum Inbewegungsetzen eines Exoskeletts (1) nach einem der Ansprüche 1 bis 7, wenn das Programm auf einem Computer ausgeführt wird.

10. Speichermedium, das von einem Computergerät gelesen werden kann, auf dem ein Computerprogrammprodukt Codeanweisungen zum Ausführen eines Verfahrens zum Inbewegungsetzen eines Exoskeletts (1) nach einem der Ansprüche 1 bis 7 umfasst.

**Claims**

1. A method for moving an exoskeleton (1) receiving a human operator, said exoskeleton (1) having a plurality of degrees of freedom including at least one degree of freedom actuated by an actuator controlled by data processing means (11) and at least one non-actuated degree of freedom, the method being **characterised in that** it comprises the implementation by the data processing means (11) of steps of:

(a) when a start request is received, generating and emitting a command to at least one of said actuators so as to put the exoskeleton (1) in a tipping state;
(b) in a database stored in data storage means (12) of triplets of:

- a set of virtual constraints on said actuated degrees of freedom, the virtual constraints being parameterised by a phase variable,
- a controller for said exoskeleton (1) capable of generating commands of said actuators so as to fulfil the virtual constraints by implementing at least one attracting stable trajectory in a topological manifold formed by all the n-tuples of the possible values for said non-actuated degrees of freedom and the phase variable,
- a stability pool formed by all the points of a hyperplane of said manifold for a given value of the phase parameter, from which the execution of said controller allows a convergence to said attracting stable trajectory;

identifying a set of virtual constraints such that said tipping state is included in said stability pool associated with this set of virtual constraints;
(c) executing the controller associated with the identified set of virtual constraints such that the exoskeleton (1) walks.

2. The method according to claim 1, wherein step (a) comprises determining a walking speed and/or direction setpoint as a function of which said command to at least one of said actuators is generated, step (c) comprising checking that said speed and/or direction setpoint is fulfilled by the current walk in said database.

3. The method according to claim 2, comprising, if said speed and/or direction setpoint is not fulfilled by the current walk, a step (d) of identifying in said database a new set of virtual constraints such that the current state of the exoskeleton (1) is included in said stability pool associated with this set of virtual constraints; and repeating step (c).

4. The method according to one of claims 2 and 3, wherein said walking speed and/or direction setpoint is determined as a function of a posture of said human operator.

5. The method according to claim 4, wherein the operator's chest is equipped with a plurality of posture sensors, said walking speed and/or direction setpoint being determined as a function of the posture of said operator's chest measured by the plurality of sensors.

6. The method according to one of claims 1 to 5, wherein the tipping state is a state in which a Zero Moment Point, ZMP, is not inside a lift surface for the exoskeleton (1).

7. The method according to one of claims 1 to 6, wherein step (c) comprises stopping the exoskeleton (1) if no acceptable set of virtual constraints is identified.

8. An exoskeleton (1) for receiving a human operator, comprising data processing means (11) and having a plurality of degrees of freedom including at least one degree of freedom actuated by an actuator controlled by the data processing means (11) and at least one non-actuated degree of freedom, **characterised in that** it comprises data storage means (12) storing a database of triplets of:

- a set of virtual constraints on said actuated degrees of freedom, the virtual constraints being parameterised by a phase variable,
- a controller for said exoskeleton (1) capable of generating commands of said actuators so as to fulfil said virtual constraints by implementing at least one attracting stable trajectory in a topological manifold formed by all the n-tuples of the possible values of said non-actuated degrees of freedom and the phase variable,
- a stability pool formed by all the points of a hyperplane of said manifold for a given value of the phase parameter, from which the execution of said controller allows a convergence to said attracting stable trajectory;

and **in that** the data processing means (11) are configured to implement:

- a module for generating and emitting a command to at least one of said actuators so as to put the exoskeleton (1) in a tipping state when a start request is received;
- a module for identifying in said database storage means (12) a set of virtual constraints such that said tipping state is included in said stability pool associated with this set of virtual constraints;
- an execution module of the controller associated with the identified set of virtual constraints such that the exoskeleton (1) walks.

9. A computer program product comprising code instructions for the execution of a method for moving an exoskeleton (1) according to one of claims 1 to 7, when said program is executed on a computer.

10. A computing equipment-readable storage means on which a computer program product comprises code instructions for the execution of a method for moving an exoskeleton (1) according to one of claims 1 to 7.

**FIG. 1**

FIG. 2

Bassin d'attraction

$\tilde{z}_i$

$\tilde{z}_j$

$\mathcal{S}$

**FIG. 3**

**FIG. 4**

## RÉFÉRENCES CITÉES DANS LA DESCRIPTION

**Documents brevets cités dans la description**

- US 7153242 B
- US 2016038371 A
- EP 2231096 A, Rex-Bionics
- WO 2015140352 A
- WO 2015140353 A

**Littérature non-brevet citée dans la description**

- **UGURLU BARKAN et al.** *Lower body exoskeleton-supported compliant bipedal walking for paraplégies: How to reduce upper body effort?*, 2014
- **KAJITA S., K. F.** Biped Walking pattern génération by using preview control of Zero-Moment Point. *ICRA,* 2003, 1620-1626